# EUROPÄISCHE PATENTSCHRIFT

(11) **EP 3 730 216 B1**
(45) Veröffentlichungstag und Bekanntmachung des Hinweises auf die Patenterteilung: **15.11.2023**
(21) Anmeldenummer: 20170994.6
(22) Anmeldetag: 23.04.2020
(51) Int. Cl.: B01L 3/00, G01N 33/543, G01N 33/68

(54) **TRÄGER UND VERFAHREN ZUM NACHWEIS EINES ANALYTEN IN DRIED BLOOD SPOTS**
HOLDER AND METHOD FOR DETECTING AN ANALYTE IN DRIED BLOOD SPOTS
SUPPORT ET PROCÉDÉ DE DÉTECTION D'UN ANALYTE DANS LES TACHES DE SANG SÉCHÉES

(30) Priorität: 25.04.2019 EP 19171226
(43) Veröffentlichungstag der Anmeldung: 28.10.2020
(73) Patentinhaber: Euroimmun Medizinische Labordiagnostika AG, 23560 Lübeck (DE)
(72) Erfinder: SEISMANN, Henning, 23863 Nienwohld (DE); WICHNER, Birgit, 23556 Lübeck (DE); WEIMANN, Alf, 23560 Lübeck (DE); HERBST, Victor, 23628 Krummesse (DE); STÖCKER, Winfried, 23627 Groß Grönau (DE)

(56) Entgegenhaltungen:
- DE-A1-102013 211 918
- US-A1- 2008 047 908
- US-A1- 2009 197 296
- US-A1- 2012 045 792

## Beschreibung

Die vorliegende Erfindung betrifft eine Verwendung eines Trägers umfassend wenigstens einen Aufnahmebereich basierend auf einem nicht gewobenen Polyolefin oder eine Verwendung des Aufnahmebereichs zur semi-quantitativen, bevorzugt quantitativen Bestimmung der Konzentration eines Analyten in einer Blutprobe, und ein Verfahren zum Nachweis eines Analyten in einer Blutprobe umfassend die Schritte Bereitstellen des Trägers, Aufbringen einer Blutprobe auf den Aufnahmebereich des Trägers, Trocknen des Trägers, Kontaktieren des Trägers mit der eingetrockneten Blutprobe mit einer Flüssigkeit, die geeignet ist, um den Analyten aus der eingetrockneten Blutprobe aufzunehmen, und Nachweisen des Analyten in der Flüssigkeit.

Für viele qualitative Bestimmungen auf dem Gebiet der Analytik und speziell der Labordiagnostik kommt es nur darauf an, dass die Anwesenheit oder Abwesenheit eines Analyten richtig festgestellt werden kann, nicht jedoch in welcher Konzentration er im Blut eines Patienten vorliegt. Das ist z.B. der Fall, wenn die Blutprobe darauf untersucht wird, ob der Patient an einer Stoffwechselkrankheit leidet, die mit einem defekten Gen assoziiert ist. Die Blutprobe muss dann lediglich darauf untersucht werden, ob ein solches Gen *vorhanden* ist, nicht jedoch wie viel von dem genetischen Material umfassend das Gen in der Probe enthalten ist.

Im Falle anderer Analyten muss festgestellt werden, in welcher Konzentration der Analyt im Blut auftritt, weil ein diagnostischer Referenz-Konzentrationsbereich bekannt ist. Liegt die Konzentration des Analyten in diesem Bereich, so spricht dies dafür, dass der Patient gesund ist. Für solche quantitativen Bestimmungen kommt es daher darauf an, dass die Menge oder Konzentration des Analyten richtig bestimmt wird.

Bei einer quantitativen Bestimmung sind sämtliche Prozessierungen des Probenmaterials zu vermeiden, bei der es zu einer Veränderung des Probenmaterials kommt, die eine Änderung der Signalhöhe bei der Messung des Analyten bedingt, die die Konzentration des Analyten anzeigt.

Es gibt zahlreiche Möglichkeiten, wie die Prozessierung einer Probe das Signal verändern kann. Abgesehen von einem möglichen Verlust des Analyten während der Prozessierung ist es auch gut möglich, dass dabei eine Veränderung beispielsweise der chemischen Beschaffenheit des Analyten eintritt, die dazu führt, dass die gleiche Menge an Analyt ein anderes Signal ergibt, was eine zu hohe oder zu niedrige Menge des Analyten anzeigt. Eine weitere Möglichkeit besteht darin, dass bei der Prozessierung ein externer chemischer Stoff zum Probenmaterial hinzugefügt oder gebildet wird, der ein ähnliches Signal bei der Messung wie der Analyt von Interesse erzeugt und das Gesamtsignal somit erhöht, oder der das Signal des Analyten unterdrückt. Schließlich ist es möglich, dass variierende Prozessierungsbedingungen eine Schwankung des gemessenen Signals bedingen.

Für die Messung von Konzentrationen bestimmter Analyten im Blut von Patienten wird dem Patient meistens venöses Blut abgenommen, gefolgt von der Aufbereitung des Blutes zu Serum. Abgesehen davon, dass dieser invasive Eingriff für den Patienten unangenehm ist und in geringem Maße sogar gesundheitliche Risiken birgt, wie das der Übelkeit oder der Ohnmacht, kann die Blutabnahme nur durch eine qualifizierte Fachkraft wie einen Arzt oder wenigstens eine Krankenschwester oder erfahrene Laborantin durchgeführt werden. Der Patient muss dazu eine Arztpraxis oder ein Krankenhaus aufsuchen.

Viel einfacher und schonender ist hingegen die Gewinnung von Kapillarblut. Nach dem Einstechen mit einem spitzen Gegenstand in die Fingerkuppe oder das Ohrläppchen des Patienten treten einige Tropfen Kapillarblut aus, die mit einer Pipette aufgenommen und auf einen absorptionsfähigen Träger gegeben werden. Sobald das Blut auf dem Träger eingetrocknet ist, kann der Träger ohne weitere Bearbeitung transportiert werden, sehr einfach sogar per Briefpost. Der Besuch der Arztpraxis wird damit überflüssig. Aus einem Träger mit eingetrocknetem Blut, bevorzugt Kapillarblut, kann ein Stück Träger mit Blut abgelöst werden. Das kann beispielsweise mit einer Vorrichtung, wie sie in der US14/900,360 beschrieben ist, oder durch Ausstanzen erreicht werden. Das abgelöste Stück Träger mit eingetrocknetem Blut wird häufig als "Dried Blood Spot" oder "DBS" bezeichnet.

Während diese Art der Probengewinnung insbesondere für qualitative Verfahren schon seit Jahren etabliert ist, zeigen sich bei quantitativen Bestimmungen einiger Analyten noch Unschärfen. Insbesondere treten bei der Bestimmung von Antikörpern aus Kapillarblut unter Verwendung herkömmlicher Träger Hintergrundsignale auf, die eine genaue Quantifizierung erschweren. Das beeinträchtigt die Verwendbarkeit von Dried Blood Spots.

Ein der vorliegenden Erfindung zu Grunde liegendes Problem ist daher die Bereitstellung von Trägern und Verfahren, die gestatten, dass eine Blutprobe, bevorzugt Kapillarblutprobe, in der Weise prozessiert und zur Bestimmung eingesetzt wird, dass das Signal, dessen Höhe die Konzentration des interessierenden Analyten anzeigt, absolut oder relativ wenigstens ausreichend mit dem Signal übereinstimmt, das man vom gleichen Patienten erhält, wenn der Analyt aus Blutserum bestimmt wird.

Ein weiteres der vorliegenden Erfindung zu Grunde liegendes Problem besteht darin, die Reproduzierbarkeit von quantitativen Bestimmungen von Analyten aus Kapillarblut durch Bereitstellung geeigneter Träger und Verfahren zu erhöhen. Unter Verwendung von Kapillarblut und dem hierin beschriebenen Träger und erfindungsgemäßen Verfahren sollen Bestimmungswerte erhalten werden, die für die Konzentration des Analyten im Blut repräsentativ sind, wie sie unter Verwendung von venösem Blut zur Bestimmung erhalten worden wären.

Ein weiteres der vorliegenden Erfindung zu Grunde liegendes Problem besteht darin, Träger und Verfahren zur quantitativen Bestimmungen von Analyten aus Kapillarblut bereitzustellen, bei der die Messung des Signals, das die Konzentration des Analyten anzeigt, mit gegenüber dem Stand der Technik verringerter oder mit tolerierbarer Störung durch Hintergrundsignale ermöglicht wird. Es ist ein zu diesem Zweck verwendbarer Träger bereitzustellen.

US9,110,053 offenbart eine Reihe von Materialien für Dried Blood Spots, nämlich cellulosebasierte Materialien, Glasfasern, Verbundmaterialien aus Glasfasern und Cellulose, Nylon, Polyester, Polypropylen, Nitrocellulose, modifizierten Polyether, Polyvinylchlorid, natürliche oder synthetische Fasern oder laminierte Materialien. Es wird darauf hingewiesen, dass cellulosefreie Glasfasern die Kapazität zur Bestimmung von small molecules erhöhen, insbesondere für pharmazeutische Analytik.

Kumar *et al.* offenbaren die Detektion von IgG zur Serodiagnostik der menschlichen Hydatidose mit Dried Blood Spots auf Filterpapier (Kumar, N., Sehgal, R., Goyal, K., and Tripathi, P. (2012) Evaluation of dried blood spots collected on filter paper for serodiagnosis of human hydatidosis by enzyme-linked immunosorbent assay, Trop. Parasitol. 2(2), 119-123, 2012).

Jamaly *et al.* offenbaren einen Test, bei dem Blutserum mit dem CAA-Antigen von Schistosoma kontaktiert wird und das aufgenommene Antigen anschließend mit einem ELISA basierend auf einem CAA-spezifischen monoklonalen Antikörper quantifiziert wird (Jamaly, S. et al., Transactions of the Royal Society of Tropical Medicine and Hygiene (1997) 91, 412-415). Bei einem Vergleich verschiedener Absorptionsmaterialien, insbesondere nicht gewobenen Polypropylenfasern mit verschiedenen Filterpapieren gelangten Jamaly *et al.* zu dem Ergebnis, dass unter Verwendung der Polypropylenfasern die höchste Ausbeute erzielt wird. Die Reproduzierbarkeit im Vergleich zu einem ELISA, bei dem Serum als Probenmaterial eingesetzt wird, wurde nicht untersucht.

In der US20080047908 A1 wird ein Träger umfassend einen Aufnahmebereich basierend auf einem nicht gewobenen Polyolefin mit eingetrocknetem Kapillarblut erwähnt.

In der DE102013211918 A1 wird eine Vorrichtung sowie ein Verfahren zur Aufbewahrung einer Körperflüssigkeitsprobe mit einem einen Grundkörper bildenden Trageelement, an dem wenigstens ein Sorptionselement, das zur Aufnahme eines Körperflüssigkeitstropfens geeignet ist, befestigt ist, erwähnt.

Die der Erfindung zu Grunde liegende Aufgabe wird durch den Gegenstand der beigefügten unabhängigen und abhängigen Ansprüche gelöst.

Hierin wird ein Träger, bevorzugt zum Nachweis eines Analyten in einer Blutprobe, umfassend wenigstens einen Aufnahmebereich basierend auf einem nicht gewobenen Polyolefin mit eingetrocknetem Kapillarblut beschrieben.

Ferner wird hierin ein Abnahmeset umfassend einen Träger, bevorzugt zum Nachweis eines Analyten in einer Blutprobe, umfassend wenigstens einen Aufnahmebereich basierend auf einem nicht gewobenen Polyolefin, eine sterile Stechhilfe zur Abnahme von Kapillarblut und optional eine Pipette beschrieben.

In einem ersten Aspekt wird das Problem gelöst durch eine Verwendung eines Trägers, bevorzugt zum Nachweis eines Analyten in einer Blutprobe, umfassend wenigstens einen Aufnahmebereich basierend auf einem nicht gewobenen Polyolefin oder Verwendung des Aufnahmebereichs zur semi-quantitativen, bevorzugt quantitativen Bestimmung der Konzentration eines Analyten in einer Blutprobe.

In einem zweiten Aspekt wird das Problem gelöst durch ein Verfahren zum Nachweis eines Analyten in einer Blutprobe umfassend die Schritte
a) Bereitstellen eines Trägers umfassend wenigstens einen Aufnahmebereich basierend auf einem nicht gewobenen Polyolefin,
b) Aufbringen einer Blutprobe auf den Aufnahmebereich,
c) Trocknen des Trägers,
d) Kontaktieren des Trägers oder des Aufnahmebereichs davon umfassend die eingetrocknete Blutprobe aus Schritt c) mit einer Flüssigkeit, die geeignet ist, um den Analyten aus der eingetrockneten Blutprobe aufzunehmen, und
e) Nachweisen des Analyten in der Flüssigkeit.
wobei der Analyt bevorzugt ein Antikörper ist und der Nachweis oder die Bestimmung des Analyten mit einem diagnostisch nützlichen Träger umfassend ein Mittel zum spezifischen Einfangen des Antikörpers durchgeführt wird.

In einer bevorzugten Ausführungsform ist der wenigstens eine Aufnahmebereich von einem Material mit geringerer Absorptionsfähigkeit als der des Aufnahmebereiches umgeben, wobei das Material bevorzugt Pappe ist.

In einer bevorzugten Ausführungsform umfasst der Träger wenigstens zwei Aufnahmebereiche basierend auf einem nicht gewobenen Polyolefin, die voneinander getrennt sind, bevorzugt durch einen Bereich basierend auf einem Material mit geringerer Absorptionsfähigkeit als das der Aufnahmebereiche, wobei das Material bevorzugt Pappe ist. In einer bevorzugten Ausführungsform handelt es sich um einen Träger umfassend wenigstens einen Aufnahmebereich basierend auf einem nicht gewobenen Polyolefin mit eingetrocknetem Kapillarblut zur Bereitstellung einer Probe für den Nachweis eines darin enthaltenen Analyten.

In einer weiter bevorzugten Ausführungsform handelt es sich um die Verwendung eines Trägers, der wenigstens einen Aufnahmebereich basierend auf einem nicht gewobenen Polyolefin umfasst, wobei der wenigstens eine Aufnahmebereich eingetrocknetes Kapillarblut als aufgenommene Blutprobe umfasst, zur Bereitstellung einer Probe für den Nachweis eines darin enthaltenen Analyten.

In einer bevorzugten Ausführungsform handelt es sich bei der Blutprobe um Kapillarblut.

In einer bevorzugten Ausführungsform handelt es sich bei dem Kapillarblut um unbehandeltes Kapillarblut.

In einer bevorzugten Ausführungsform handelt es sich bei dem Polyolefin um Polypropylen.

In einer bevorzugten Ausführungsform ist der Nachweis ein semi-quantitativer, bevorzugt ein quantitativer Nachweis. In einer weiteren bevorzugten Ausführungsform ist der Nachweis ein qualitativer Nachweis.

In einer bevorzugten Ausführungsform dient die Verwendung oder das Verfahren der Diagnose einer Lebensmittelallergie oder -unverträglichkeit.

In einer bevorzugten Ausführungsform wird der Nachweis oder die Bestimmung mit einem diagnostisch nützlichen Träger umfassend ein Mittel zum spezifischen Einfangen eines Antikörpers durchgeführt.

In einer bevorzugten Ausführungsform ist der diagnostisch nützliche Träger aus der Gruppe ausgewählt, die einen Blot; bevorzugt Lineblot, Dotblot oder Westernblot; einen Bead, eine Mikrotiterplatte, einen Teststreifen, einen Mikroarray, einen Glasträger, einen Biochip und eine Lateral Flow-Vorrichtung umfasst.

In einer bevorzugten Ausführungsform handelt es sich bei dem Analyten um einen Antikörper, bevorzugt um einen Antikörper der IgG-Klasse.

In einer bevorzugten Ausführungsform handelt es sich bei dem Antikörper um einen Antikörper gegen ein allergenhaltiges und/oder proteinhaltiges Material basierend auf einem Nahrungsmittel, bevorzugt aus der Gruppe, die Zucchini, Paprika, Hühnerfleisch, Kabeljau, Lachs, Walnuss, Wassermelone, Kiwi, Perlhuhn, Kaninchen, Krabbe, Rotbarsch, Schnittlauch und Papaya umfasst.

Die vorliegende Erfindung basiert auf der überraschenden Erkenntnis der Erfinder, dass nicht gewobene Polyolefinfasern als Mittel zur Aufnahme einer wässrigen Probe, die dann darauf eintrocknet, nach der Aufnahme des Materials in ein anderes wässriges Medium die vorteilhafte Eigenschaft aufweist, dass Analyten aus der wässrigen Probe mit einem Ergebnis quantitativ bestimmt werden können, das mit dem aus einer Serumprobe direkt bestimmten besonders genau übereinstimmt. Dies betrifft insbesondere die quantitative Bestimmung von Antikörpern als Analyt.

In einer bevorzugten Ausführungsform wird unter dem Begriff "Analyt", wie hierin verwendet, ein in der Blutprobe vorhandener Stoff verstanden, der beim Eintrocknen der Blutprobe auf dem Aufnahmebereich des Trägers verbleibt und von dort für die quantitative Bestimmung in eine andere Flüssigkeit überführt werden kann. Besonders bevorzugt sind in wässrigen Lösungen gut lösliche Stoffe, die in der Blutprobe aufgelöst waren. Möglich sind jedoch auch Stoffe wie feste Partikel, die in der Lösung in Form einer Suspension vorliegen. Auch ihre Konzentration in der Blutprobe oder einer wässrigen Lösung kann mit geeigneten physikalischen Messverfahren wie dem Nachweis von Lichtstreuung quantitativ bestimmt werden. In einer besonders bevorzugten Ausführungsform ist der Analyt aus der Gruppe ausgewählt, die einen Metaboliten, ein Protein, eine Nukleinsäure und ein Lipid umfasst und ist besonders bevorzugt ein Antikörper, noch bevorzugter ausgewählt aus der Gruppe von Klassen, die IgA, IgM, IgG und IgE umfassen, am bevorzugtesten IgG. In einer bevorzugten Ausführungsform handelt es sich bei dem Antikörper um einen Antikörper von einem Säugetier, noch bevorzugter von einem Menschen.

In einer bevorzugten Ausführungsform handelt es sich bei der Probe um eine Probe von einem Säugetier, noch bevorzugter von einem Menschen.

Bei dem Träger handelt es sich um einen festen Träger, der einen Aufnahmebereich aufweist. Der Aufnahmebereich umfasst ein Material, das eine wässrige Blutprobe absorbiert, und basiert auf Polyolefin, bevorzugt umfasst nicht gewobenes Polyolefin mit einem Massenanteil von wenigstens 5, 10, 20, 30, 40, 50, 60, 70, 80, 90 oder 95%. Der Aufnahmebereich ist so beschaffen und von anderen absorptionsfähigen Teilen des Trägers getrennt, dass eine räumliche Ausbreitung der aufgetropften Blutprobe über den Träger begrenzt wird. Er ist bevorzugt unmittelbar geometrisch zugänglich für das Auftropfen der Blutprobe. Für den Fall, dass der Träger zwei, drei, vier, sechs, acht, 10, 12, 20, 24, 48 oder mehr Aufnahmebereiche aufweist, sind diese bevorzugt derart voneinander abgetrennt, dass eine in einen Aufnahmebereich getropfte Blutprobe nicht andere Aufnahmebereiche kontaminieren kann, besonders für den Fall, dass mehrere Blutproben von mehr als einem Patienten aufgetropft werden. Ein Aufnahmebereich ist bevorzugt von einem hydrophoben Material und/oder einem Material mit hydrophoben Eigenschaften seitlich umgeben, und noch bevorzugter auch auf seiner Unterseite, d.h. bevorzugt der Seite, bei der es sich *nicht* um die Seite handelt, von der die Blutprobe hinzugegeben wird. Der Träger kann ein beschreib- oder bedruckbares Feld umfassen, um Daten des Patienten und die gewünschte Untersuchung der Probe zu vermerken. Er kann weiterhin einen maschinenlesbaren Code umfassen, z.B. einen Barcode. Der Aufnahmebereich ist bevorzugt so beschaffen, dass er zusammen mit einem erheblichen Teil der eingetrockneten Blutprobe leicht von dem Rest des Trägers abgelöst werden kann, beispielsweise durch Abtrennung vom Rest in Form einer geschwächten Linie wie einer Perforation oder dadurch, dass er über einen abreißbaren Steg mit dem Rest des Trägers verbunden ist.

Das Polyolefin ist bevorzugt aus der Gruppe ausgewählt, die Polyethylen, Polypropylen, Polymethylpenten, Polyisobutylen und Polybutylen umfasst, und ist bevorzugt Polypropylen.

Das Abnehmset umfasst neben dem Träger bevorzugt eine Stechhilfe zur Abnahme von Kapillarblut, die bevorzugt steril ist, und eine Pipette, bei der es sich bevorzugt um eine Pipette mit den Ausmaßen handelt, dass einige Tropfen Blut eingeführt und auf den Träger wieder abgegeben werden können.

In einer bevorzugten Ausführungsform wird unter dem Begriff "quantitative Bestimmung" eine Bestimmung verstanden, die eine Aussage über die absolute Konzentration des Analyten gestattet, noch bevorzugter mit einem numerischen Wert. Es kann sich um eine semi-quantitative Bestimmung handeln, bei der eine Zuordnung der Konzentration zu einem Bereich aus wenigstens drei, möglichst vier Konzentrationsbereichen ermöglicht wird, z.B. negativ, schwach positiv und positiv, oder eine relative Konzentrationsbestimmung. Insbesondere erfolgt die Konzentrationsbestimmung mit Hilfe von Kalibratoren, wobei es sich bevorzugt um zwei oder mehr, bevorzugt vier Einheiten, bevorzugt Lösungen oder auf einem diagnostisch nützlichen Träger gecoateten festen Analyten handelt, die jeweils eine bekannte Menge des Analyten enthalten, wobei die zwei oder mehr Einheiten jeweils eine andere bekannte Menge umfassen.

In einer bevorzugten Ausführungsform wird unter dem Begriff "absorptionsfähig", wie hierin verwendet, verstanden, dass das so bezeichnete Material in der Lage ist, einen Bluttropfen aufzunehmen, wobei der Wasseranteil zunächst aufgenommen und anschließend beim Trocknen an die Umgebung abgegeben wird, wobei die darin gelösten Komponenten, darunter der Analyt wie ein Antikörper, im Material verbleiben, und durch erneutes Kontaktieren mit einem geeigneten Lösungsmittel, bevorzugt einem wässrigen Puffer, wie herausgelöst werden können. In einer bevorzugten Ausführungsform besteht der Aufnahmebereich aus nicht gewobenem Polyolefin, abgesehen davon, dass handelsübliche Verunreinigungen oder Zusatzstoffe anwesend sein können.

Bevorzugt wird die quantitative Bestimmung mit einem Verfahren ausgeführt, das aus der Gruppe ausgewählt ist, die Immundiffusion, Immunelektrophorese, Lichtstreuung, Agglutination und Immuntest mit Markierung - wie die aus der Gruppe umfassend Immuntest mit radioaktiver Markierung, mit enzymatischer Markierung, bevorzugter ELISA, mit Chemilumineszenz-Markierung, bevorzugter Elektrochemilumineszenz-Markierung und mit Immunfluoreszenz-Markierung, bevorzugter indirekte Immunfluoreszenz-Markierung - umfasst, bevorzugt mit ELISA.

Das erfindungsgemäße Verfahren betrifft das Bereitstellen des hierin beschriebenen Trägers, gefolgt von dem Aufbringen einer Blutprobe, gefolgt vom Trocknen des Trägers, was durch Inkubation bei Raumtemperatur für einige Minuten, bevorzugt mehrere Stunden oder über Nacht geschehen kann. Alternativ wird ein Träger bereitgestellt, der bereits eine eingetrocknete Blutprobe aufweist. Erfindungsgemäß kann der Aufnahmebereich oder ein Teil davon mit wenigstens einem Teil der eingetrockneten Blutprobe für die weiteren Schritte verwendet werden. Dafür kann diese aus dem Träger abgelöst werden, bevorzugt durch Druckausüben über eine perforierte Linie, durch Ausstanzen oder durch Abreißen. Es sind auch Vorrichtungen bekannt, mit denen ein Träger mit mehreren, nicht abgelösten Aufnahmebereichen im Folgenden derart mit mehreren Portionen einer Flüssigkeit oder mehreren Flüssigkeiten kontaktiert wird, dass eine Kreuzkontamination der verschiedenen Proben vermieden werden kann, wie beispielsweise in der US2012/0125127 beschrieben. In einer besonders bevorzugten Ausführungsform handelt es sich bei dem Blut um Blut von einem Patienten.

In einer besonders bevorzugten Ausführungsform wird der Schritt des Trocknens derart durchgeführt, dass ausschließlich wässrige, flüchtige Komponenten der Probe, besonders bevorzugt aus dem Kapillarblut, aus dem Aufnahmebereich des Trägers entweichen, wohingegen nicht-flüchtige Komponenten, beispielsweise Proteine, bevorzugt Antikörper, Salze und ähnliche Verbindungen auf dem Träger im trockenen Zustand verbleiben. Insbesondere in der Probe vorhandenes Wasser wird von den darin gelösten Analyten getrennt und es wird entfernt. Trennungsschritte wie das Zentrifugieren, bei denen sowohl flüssige als auch nicht-flüssige Komponenten entnommen, aber nicht voneinander getrennt werden, stellen keinen Trocknungsschritt dar. Optional kann der wenigstens eine Aufnahmebereich nach dem Trocknen vom Träger abgetrennt werden. Es besteht die Möglichkeit, ihn anschließend zu verschicken, z.B. mit Hilfe eines Kuriers oder per Post. Aus dem Aufnahmebereich auf dem Träger, auf dem die Blutprobe eingetrocknet ist, wird sie durch Kontaktieren des Aufnahmebereichs oder des Trägers mit einer Flüssigkeit eluiert, die geeignet ist, um den Analyten aus der eingetrockneten Blutprobe aufzunehmen. Dabei kommen insbesondere wässrige Puffer mit geeignetem pH und Salzgehalt in Frage, beispielsweise PBS. Die genaue Zusammensetzung der Flüssigkeit und die Bedingungen und die Dauer des Kontaktierens können durch Routineversuche zur Stabilisierung und Optimierung zwecks möglichst vollständiger Aufnahme des Analyten in die Flüssigkeit herausgefunden werden und hängen von der Natur des Analyten ab. Die Flüssigkeit wird möglichst auch gleich derart gewählt, dass sie mit dem nachfolgend eingesetzten Verfahren zum Nachweisen des Analyten kompatibel ist.

Anschließend wird der Analyt von Interesse in der Flüssigkeit nachgewiesen. Dabei wird bestimmt, ob der Analyt anwesend oder abwesend bzw. in einer Konzentration oberhalb der Nachweisgrenze der eingesetzten Nachweismethode vorhanden ist. Bevorzugt wird der Analyt semiquantitativ oder quantitativ nachgewiesen.

Verschiedene Optionen zur Durchführung des Verfahrens sind im Stand der Technik beschrieben, z.B. Grüner, N., Stambouli, O. und Ross, R. S. (2015) Dried Blood Spots - Preparing and Processing for Use in Immunoassays and in Molecular Techniques, J. Vis. Exp 97, 52619.

Bevorzugt ist ein Aufnahmebereich von einem anderen Aufnahmebereich über einen Bereich basierend auf einem Material getrennt, das eine geringere Absorptionsfähigkeit aufweist als der Aufnahmebereich. In einer bevorzugten Ausführungsform bedeutet der Begriff "Absorptionsfähigkeit", wie hierin verwendet, die Fähigkeit, relativ zur Masse ein möglichst hohes Volumen einer Flüssigkeit, bevorzugt Kapillarblut oder Wasser, noch bevorzugter Kapillarblut, aufzunehmen. Sie kann bevorzugt dadurch gemessen werden, dass zu zwei gleich schweren Stücken der zwei zu vergleichenden Materialien gleiche Volumina der Flüssigkeit unter gleichen Bedingungen gegeben werden, bis eines der Materialien die Flüssigkeit nicht mehr vollständig aufnimmt.

Der Nachweis wird bevorzugt mit einem diagnostisch nützlichen Träger umfassend ein Mittel zum spezifischen Einfangen des Antikörpers durchgeführt. Bei einem Bead als Träger kann es sich um einen magnetischen oder fluoreszenten Bead handeln.

In einer bevorzugten Ausführungsform wird unter dem "Mittel zum spezifischen Einfangen eines Antikörpers", wie hierin verwendet, ein Reagenz, Antigen, Peptid, Protein, Peptidomimetikum oder ähnliches Molekül verstanden, das mit ausreichender Bindungsstärke an den zu bestimmenden Antikörper bindet, wobei bevorzugter andere Antikörper nicht in erheblichem Maß gebunden werden und in der Lösung verbleiben. Noch bevorzugter ist es, wenn die Bindung stärker als eine Bindungsreaktion ist, die durch eine Dissoziationskonstante gekennzeichnet ist, die 1 × 10⁻⁵ M, bevorzugter 1 × 10⁻⁷ M, bevorzugter 1 × 10⁻⁸ M, bevorzugter 1 × 10⁻⁹ M, bevorzugter 1 × 10⁻¹⁰ M, bevorzugter 1 × 10⁻¹¹ M, bevorzugter 1 × 10⁻¹² M beträgt, wie durch Surface Plasmon Resonanz unter Verwendung von Biacore-Ausrüstung bei 25°C in PBS Buffer bei pH 7 bestimmt wird. Der zu bestimmende Antikörper kann ein Antikörper spezifisch gegen ein bestimmtes Antigen oder eine bestimmte Klasse von Antikörpern binden, bevorzugt aus der Gruppe umfassend IgM, IgG, IgA oder IgE, am bevorzugtesten IgG. Im letzteren Fall handelt es sich bei dem Mittel bevorzugt um ein Mittel, das gegen die konstante Region der einzufangenden Klasse von Antigenen handelt. Entsprechende Mittel sind dem Fachmann bekannt und umfassen sekundäre Antikörper, Protein A und Protein G.

Der Gegenstand der vorliegenden Erfindung dient insbesondere dem einfachen, dabei jedoch gegenüber dem Stand der Technik verlässlicheren Nachweis von IgG-Antikörpern gegen Nahrungsmittelbestandteile, insbesondere, was die Übereinstimmung einer semi-quantitativen oder quantitativen Bestimmung von IgG aus einer Probe mit Kapillarblut gegenüber einer semi-quantitativen oder quantitativen Bestimmung von IgG aus einer Probe mit Vollblut oder Serum, bevorzugt Serum angeht. Damit wird unter Verwendung des Gegenstandes der vorliegenden Erfindung ein zuverlässigerer Hinweis auf Nahrungsmittelunverträglichkeiten und damit assoziierte Beschwerden erhalten. Es ist nicht erforderlich, dass prozessiertes oder mit Zusätzen versehenes Blut verwendet wird. Insbesondere muss die Blutprobe kein Heparin enthalten.

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

### Beispiel:

### Gewinnung des Probenmaterials:

Vollblut wurde auf unterschiedliche Membranen (Ahlstrom 226 - Ahlstrom-Munksjö, auch bezeichnet als "Standard Membran"; Porex BNW441435 - Porex Filtration Group, auch bezeichnet als "neue Membran") getropft und mindestens vier Stunden lang bei Raumtemperatur getrocknet. Die Membranen wurden mit Trockenmittel in Siegelrandbeuteln bei 4 °C gelagert und vor jeder weiteren Verwendung auf Raumtemperatur äquilibriert.

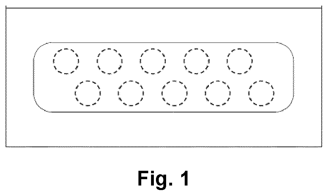

In **Fig. 1** ist schematisch ein Träger abgebildet, der aus einer der oben beschriebenen Membranen mit markierten Probenauftragsbereichen besteht, die auf einen Pappträger aufgebracht ist. In den Bereich der markierten Kreise wird das Vollblut aufgetropft.

### Extraktion des Probenmaterials:

Es wurden jeweils Stanzen mit einer Größe von 3/16 Inch aus dem Bereich der in Figur 1 markierten Kreise, d.h. aus dem Bereich mit den oben beschriebenen Membranen mit dem aufgebrachten und getrockneten Vollblut, in einem 1,5 ml Schraubdeckelröhrchen mit 1250 µl Universalpuffer (Sarstedt AG; 1,5 ml Mikroröhre Protein LB; Art.Nr. 72.694.600) versehen und eine Stunde lang bei Raumtemperatur unter Schütteln (Orbitalschüttler; 400 rpm) inkubiert und dann die Flüssigkeit in ein neues Probenröhrchen überführt.

### Überführung des extrahierten Probenmaterials auf EUROLine IgG Food Teststreifen:

Für die Inkubation mit EUROLine IgG Food Teststreifen (Euroimmun Medizinische Labordiagnostika AG; **DP 3022-0802-1 G; DP 3022-0802-2 G; DP 3024-0404-1 G; DP 3024-0404-2 G)** wurden 510 µl des jeweils über eine der Membranen erhaltenen extrahierten Probenmaterials auf den zuvor nach Herstellerangaben äquilibrierten Teststreifen gegeben. Die weitere Inkubation erfolgte gemäß der Testanleitung.

### Inkubation der EUROLine IgG Food Teststreifen und Vergleich der Probenmaterialien:

Zur Überprüfung der Vergleichbarkeit von den Probenmaterialien, die über die beiden DBS-Verfahren gewonnen wurden, mit dem Standard-Probenmaterial (Serum) wurden die EUROLine IgG Food Teststreifen mit den beiden DBS-Probenmaterialien (einmal erhalten über die "Standard Membran" und einmal erhalten über die "neue Membran"), jeweils vom gleichen Patienten erhalten, gemäß Testanleitung bzw. wie oben beschrieben inkubiert. Das Standard-Probenmaterial (Serum), jeweils vom gleichen Patienten, wurde ebenfalls gemäß Testanleitung inkubiert.

Die Validierung wurde mit der EUROLineScan Software durchgeführt und die Ergebnisse auf Basis der ermittelten Klassenergebnisse (Klasse 0-4), bzw. auf Basis der ermittelten Bandenintensitäten verglichen.

### Ergebnis:

Die Rohdaten aus diesem Versuch sind in **Tab. 1** abgebildet, wobei alle Werte markiert sind, die mit der Standard Membran ein positives Ergebnis geben, in der Serumprobe und des DBS-Probe mit der neuen Membran aber ein negatives.

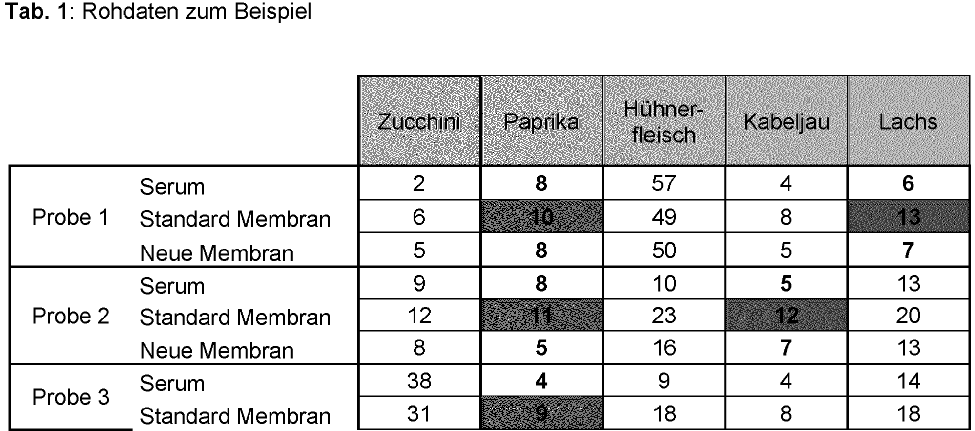

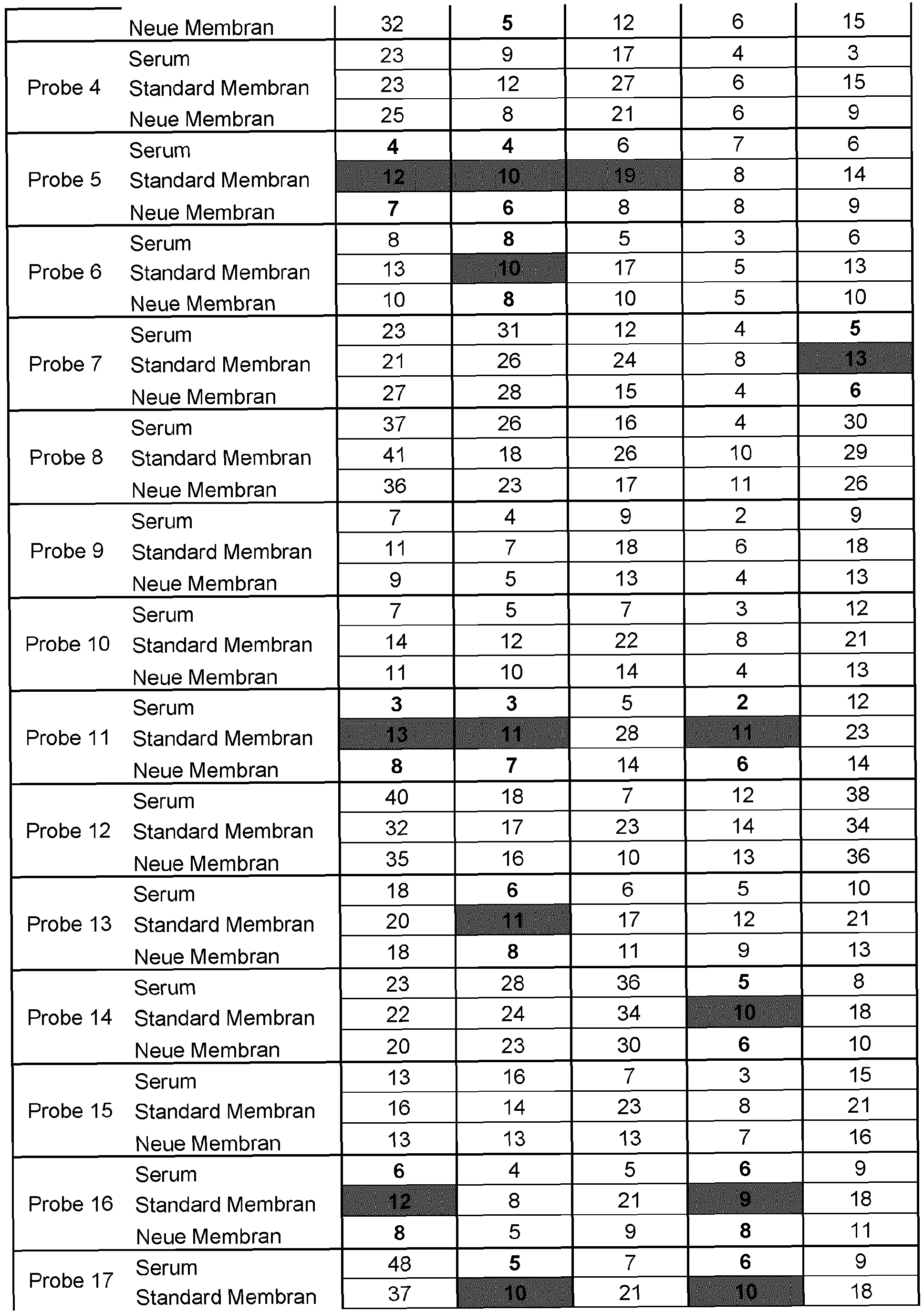

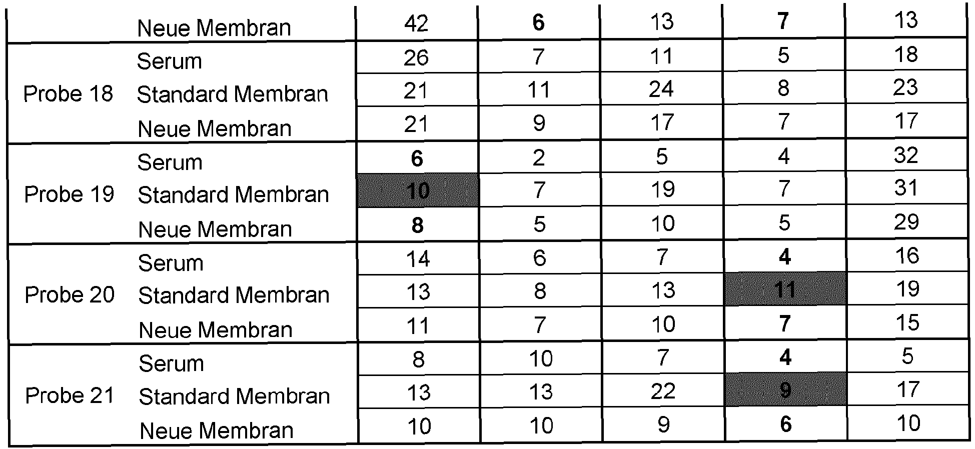

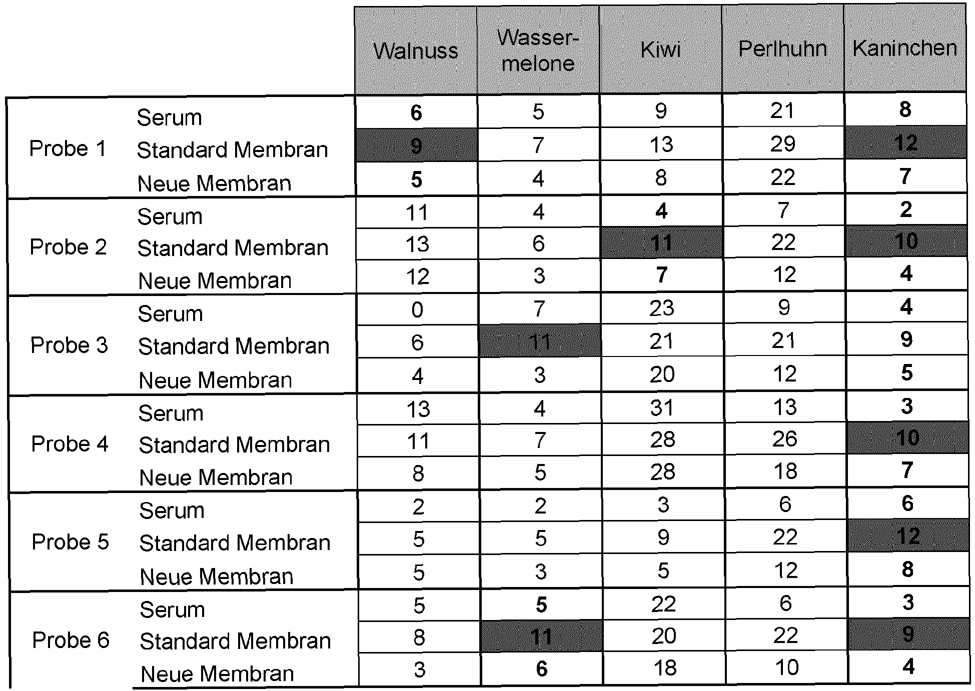

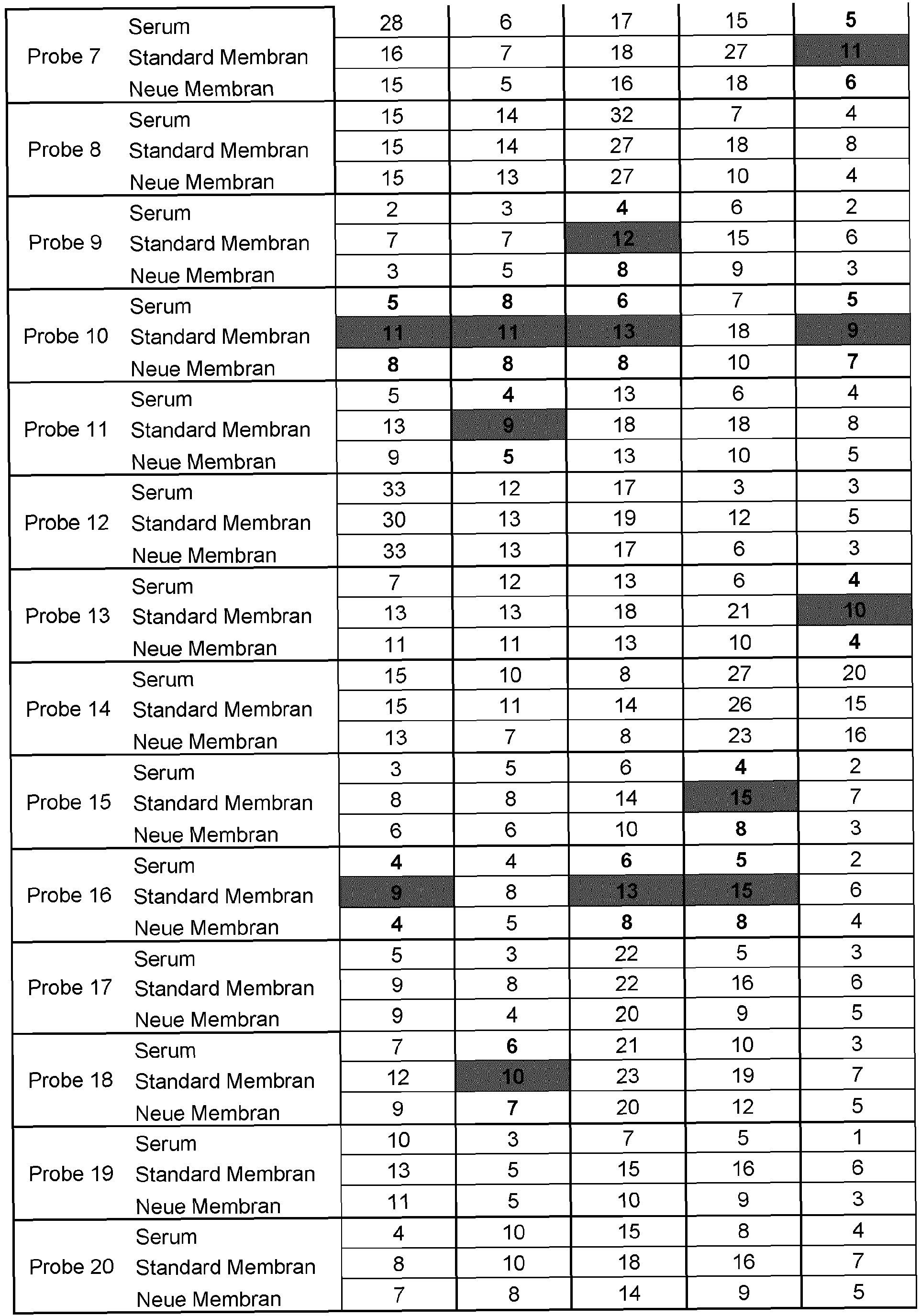

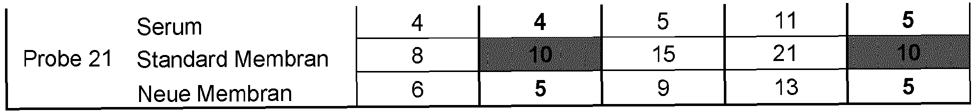

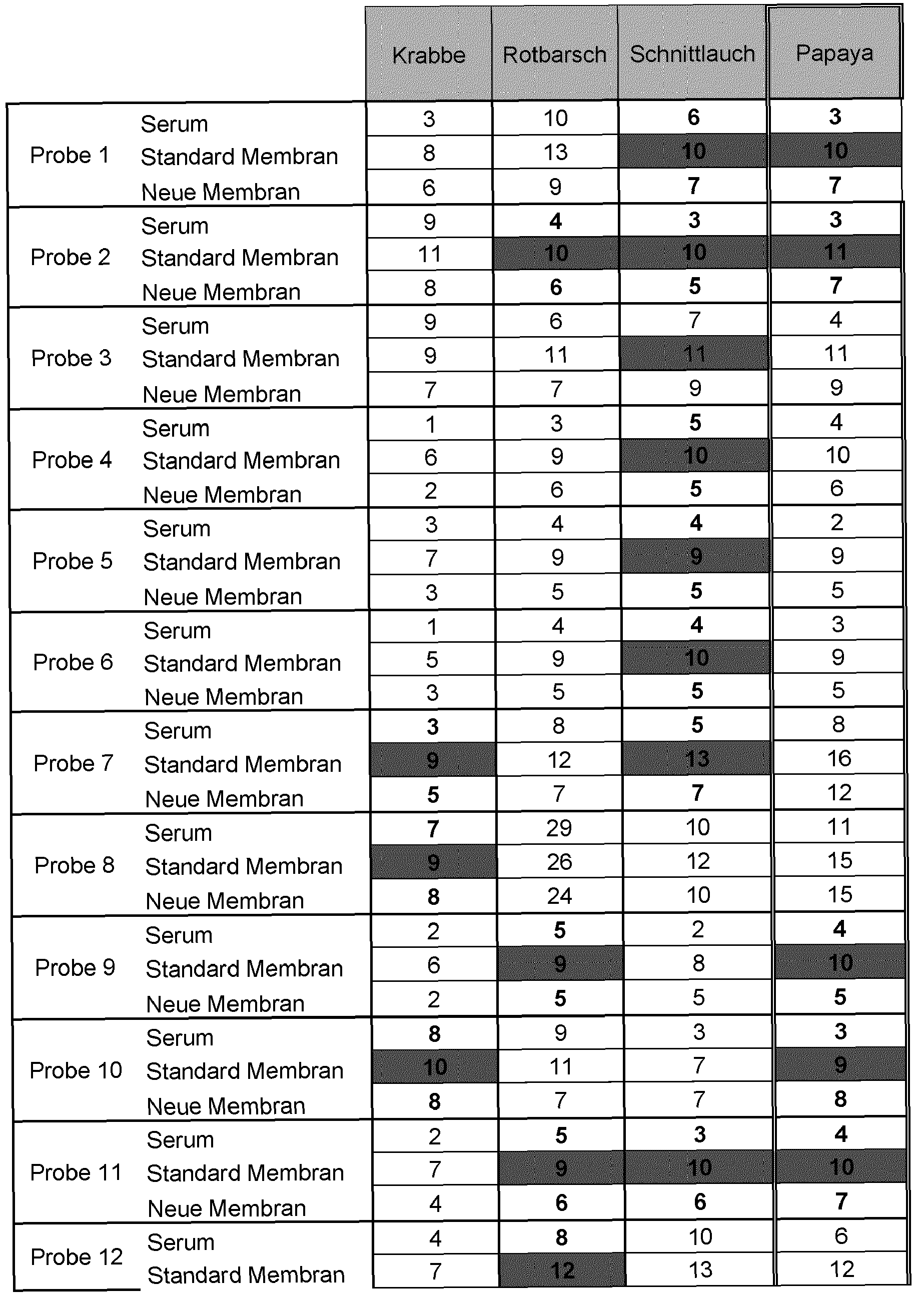

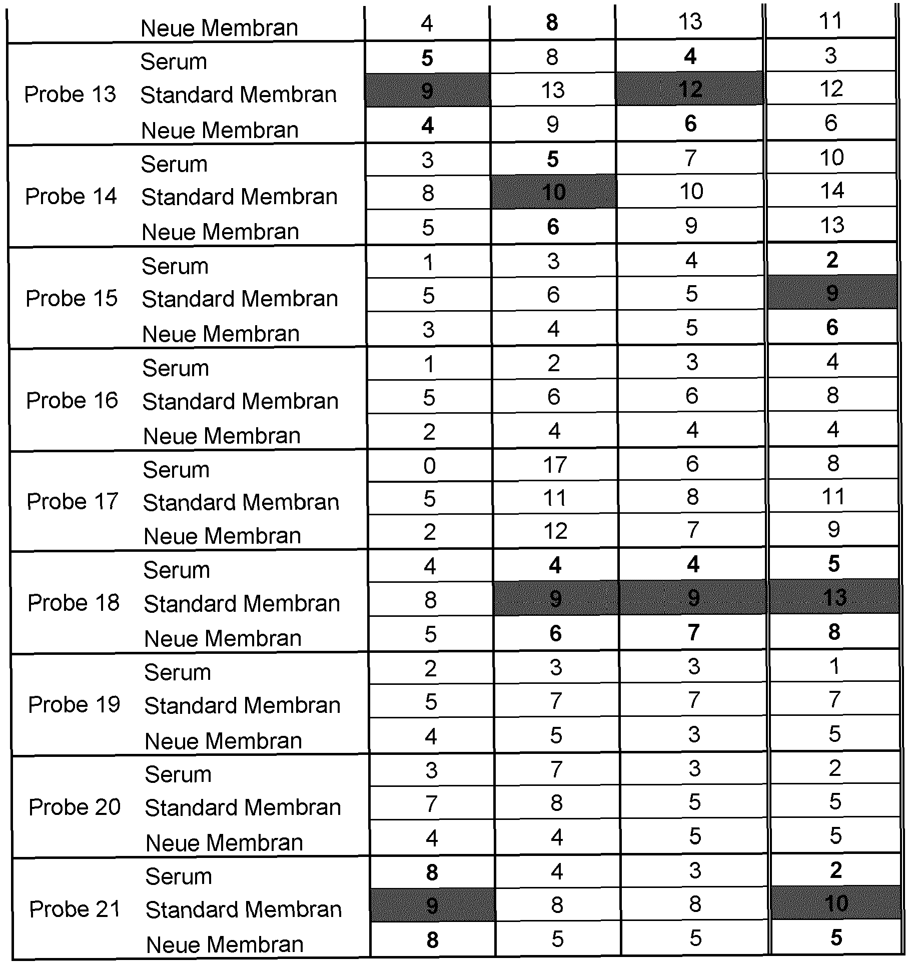

| | | Huhnereiweib | Kuhmilch | Summe intensitat uber alle Parameter pro Probe |
|---|---|---|---|---|
| Probe 1 | Serum | 75 | 129 | 352 |
| | Standard Membran | 67 | 125 | 389 |
| | Neue Membran | 69 | 126 | 345 |
| Probe 2 | Serum | 67 | 52 | 211 |
| | Standard Membran | 57 | 42 | 281 |
| | Neue Membran | 63 | 50 | 226 |
| Probe 3 | Serum | 65 | 15 | 218 |
| | Standard Membran | 51 | 7 | 252 |
| | Neue Membran | 57 | 11 | 214 |
| Probe 4 | Serum | 59 | 58 | 250 |
| | Standard Membran | 49 | 41 | 290 |
| | Neue Membran | 60 | 52 | 266 |
| Probe 5 | Serum | 3 | 26 | 88 |
| | Standard Membran | 8 | 14 | 172 |
| | Neue Membran | 5 | 19 | 113 |
| Probe 6 | Serum | 117 | 25 | 225 |
| | Standard Membran | 108 | 13 | 282 |
| | Neue Membran | 114 | 20 | 236 |
| Probe 7 | Serum | 69 | 80 | 319 |
| | Standard Membran | 62 | 60 | 343 |
| | Neue Membran | 70 | 76 | 317 |
| Probe 8 | Serum | 105 | 80 | 427 |
| | Standard Membran | 94 | 52 | 414 |
| | Neue Membran | 97 | 65 | 401 |
| Probe 9 | Serum | 31 | 6 | 98 |
| | Standard Membran | 21 | 2 | 163 |
| | Neue Membran | 27 | 4 | 120 |
| Probe 10 | Serum | 56 | 22 | 166 |
| | Standard Membran | 63 | 17 | 256 |
| | Neue Membran | 71 | 22 | 216 |
| Probe 11 | Serum | 60 | 40 | 171 |
| | Standard Membran | 68 | 37 | 293 |
| | Neue Membran | 73 | 44 | 231 |
| Probe 12 | Serum | 84 | 40 | 335 |
| | Standard Membran | 68 | 23 | 334 |
| | Neue Membran | 81 | 36 | 335 |
| Probe 13 | Serum | 18 | 58 | 183 |
| | Standard Membran | 18 | 40 | 260 |
| | Neue Membran | 18 | 50 | 201 |
| Probe 14 | Serum | 78 | 19 | 302 |
| | Standard Membran | 63 | 10 | 304 |
| | Neue Membran | 65 | 12 | 266 |
| Probe 15 | Serum | 77 | 21 | 182 |
| | Standard Membran | 58 | 12 | 229 |
| | Neue Membran | 62 | 15 | 190 |
| Probe 16 | Serum | 59 | 9 | 129 |
| | Standard Membran | 47 | 5 | 196 |
| | Neue Membran | 49 | 7 | 140 |
| Probe 17 | Serum | 81 | 35 | 260 |
| | Standard Membran | 72 | 20 | 284 |
| | Neue Membran | 79 | 28 | 265 |
| Probe 18 | Serum | 56 | 98 | 285 |
| | Standard Membran | 47 | 83 | 327 |
| | Neue Membran | 54 | 88 | 292 |
| Probe 19 | Serum | 83 | 7 | 174 |
| | Standard Membran | 74 | 4 | 233 |
| | Neue Membran | 74 | 5 | 191 |
| Probe 20 | Serum | 90 | 30 | 223 |
| | Standard Membran | 69 | 14 | 231 |
| | Neue Membran | 78 | 17 | 206 |
| Probe 21 | Serum | 73 | 17 | 170 |
| | Standard Membran | 57 | 8 | 238 |
| | Neue Membran | 70 | 14 | 190 |

| | Summe Intensität über alle Parameter pro Probenmaterial | Abweichung [%] Gesamtintensität im Vergleich zu Serum |
|---|---|---|
| Serum | 4768 | |
| Standard Membran | 5771 | 17,38 |
| Neue Membran | 4961 | 3,89 |

Im Vergleich der beiden unterschiedlichen Materialien, die für die Gewinnung der DBS verwendet wurden, zeigte sich bei der Ahlstrom 226 Membran, bei der es sich um ein Filterpapier auf Basis von 100% Baumwolle handelt, bei den meisten Parametern unabhängig von der Probe ein deutlich erhöhtes Hintergrundsignal, welches zu falsch positiven Ergebnissen führte. Lediglich bei den zwei Parametern Hühnereiweiß und Kuhmilchtrat dieses Problem nicht auf.

Über alle Proben gemittelt weichen die mit der "Standard-Membran" erhaltenen Werte um mehr als 15 % von den Werten ab, die mit Serumproben erhalten wurden.

Ähnliche Ergebnisse wurden mit derWhatman 903-Membran, einer weiteren Memran für DBS-Anwendungen, erhalten, die ebenfalls auf Baumwolle basiert. Die Daten dazu werden hier nicht gezeigt.

Deutlich bessere Ergebnisse konnten unter Verwendung der "neuen Membran" (Porex BNW441435) erzielt werden, die auf nicht gewobenen Polypropylen-Fasern basiert. Die mit dieser Membran erhaltenen Werte weichen im Mittel nur um weniger als 4 Prozent von den mit Serum erhaltenen Werten ab.

## Patentansprüche

1. Verwendung eines Trägers umfassend wenigstens einen Aufnahmebereich basierend auf einem nicht gewobenen Polyolefin oder Verwendung des Aufnahmebereichs zur semi-quantitativen, bevorzugt quantitativen Bestimmung der Konzentration eines Analyten in einer Blutprobe.

2. Verfahren zum Nachweis eines Analyten in einer Blutprobe umfassend die Schritte
a) Bereitstellen eines Trägers umfassend wenigstens einen Aufnahmebereich basierend auf einem nicht gewobenen Polyolefin,
b) Aufbringen einer Kapillarblutprobe auf den Aufnahmebereich,
c) Trocknen des Trägers,
d) Kontaktieren des Trägers oder des Aufnahmebereichs davon umfassend die eingetrocknete Blutprobe aus Schritt c) mit einer Flüssigkeit, die geeignet ist, um den Analyten aus der eingetrockneten Blutprobe aufzunehmen, und
e) Nachweisen des Analyten in der Flüssigkeit.

3. Verwendung oder Verfahren nach Anspruch 1 oder 2, wobei der wenigstens eine Aufnahmebereich von einem Material mit geringerer Absorptionsfähigkeit als das des Aufnahmebereiches, bevorzugt Pappe, umgeben ist.

4. Verwendung oder Verfahren nach einem der Ansprüche 1 bis 3, wobei der Träger wenigstens zwei Aufnahmebereiche basierend auf einem nicht gewobenen Polyolefin umfasst, die voneinander getrennt sind, bevorzugt durch einen Bereich basierend auf einem Material mit geringerer Absorptionsfähigkeit als das des Aufnahmebereiches, bevorzugt Pappe.

5. Verwendung oder Verfahren nach einem der Ansprüche 1 bis 4, wobei es sich bei der Blutprobe um Kapillarblut handelt.

6. Verwendung oder Verfahren nach einem der Ansprüche 1 bis 5, wobei es sich bei dem Kapillarblut um unbehandeltes Kapillarblut handelt.

7. Verwendung oder Verfahren nach einem der Ansprüche 1 bis 6, wobei es sich bei dem Polyolefin um Polypropylen handelt.

8. Verwendung oder Verfahren nach einem der Ansprüche 1 bis 7, wobei der Nachweis ein semi-quantitativer, bevorzugt ein quantitativer Nachweis ist.

9. Verwendung oder Verfahren nach einem der Ansprüche 1 bis 8, wobei der Nachweis oder die Bestimmung mit einem diagnostisch nützlichen Träger umfassend ein Mittel zum spezifischen Einfangen eines Antikörpers durchgeführt wird.

10. Verwendung oder Verfahren gemäß Anspruch 9, wobei der diagnostisch nützliche Träger aus der Gruppe ausgewählt ist, die einen Blot; bevorzugt Lineblot, Dotblot oder Westernblot; einen Bead, eine Mikrotiterplatte, einen Teststreifen, einen Mikroarray, einen Glasträger, einen Biochip und eine Lateral Flow-Vorrichtung umfasst.

11. Verwendung oder Verfahren nach einem der Ansprüche 1 bis 10, wobei es sich bei dem Analyten um einen Antikörper handelt, bevorzugt um einen Antikörper der IgG-Klasse.

12. Verwendung oder Verfahren nach Anspruch 11, wobei es sich bei dem Antikörper um einen Antikörper gegen ein allergenhaltiges und/oder proteinhaltiges Material basierend auf einem Nahrungsmittel handelt, bevorzugt aus der Gruppe, die Zucchini, Paprika, Hühnerfleisch, Kabeljau, Lachs, Walnuss, Wassermelone, Kiwi, Perlhuhn, Kaninchen, Krabbe, Rotbarsch, Schnittlauch und Papaya umfasst.

## Claims

1. Use of a carrier comprising at least one take-up region based on a non-woven polyolefin or use of the take-up region for semi-quantitatively, preferably quantitatively determining the concentration of an analyte in a blood sample.

2. Method for detecting an analyte in a blood sample comprising the steps
a) providing a carrier comprising at least one take-up region based on a non-woven polyolefin,
b) applying a capillary blood sample to the take-up region,
c) drying the carrier,
d) contacting the carrier or the take-up region thereof comprising the dried-up blood sample from step c) with a liquid which is suitable for taking up the analyte from the dried-up blood sample, and
e) detecting the analyte in the liquid.

3. Use or method according to Claim 1 or 2, wherein the at least one take-up region is surrounded by a material having lower absorptivity than that of the take-up region, preferably cardboard.

4. Use or method according to any of Claims 1 to 3, wherein the carrier comprises at least two take-up regions based on a non-woven polyolefin that are separated from one another, preferably by a region based on a material having lower absorptivity than that of the take-up region, preferably cardboard.

5. Use or method according to any of Claims 1 to 4, wherein the blood sample is capillary blood.

6. Use or method according to any of Claims 1 to 5, wherein the capillary blood is untreated capillary blood.

7. Use or method according to any of Claims 1 to 6, wherein the polyolefin is polypropylene.

8. Use or method according to any of Claims 1 to 7, wherein detecting is semi-quantitative detecting, preferably quantitative detecting.

9. Use or method according to any of Claims 1 to 8, wherein detecting or determining is carried out using a diagnostically useful carrier comprising a means for specifically capturing an antibody.

10. Use or method according to Claim 9, wherein the diagnostically useful carrier is selected from the group comprising a blot; preferably line blot, dot blot or western blot; a bead, a microtitre plate, a test strip, a microarray, a glass slide, a biochip and a lateral flow device.

11. Use or method according to any of Claims 1 bis 10, wherein the analyte is an antibody, preferably an antibody of the IgG class.

12. Use or method according to Claim 11, wherein the antibody is an antibody against an allergen-containing and/or protein-containing material based on a foodstuff, preferably from the group comprising courgette, pepper, chicken meat, codfish, salmon, walnut, watermelon, kiwi fruit, guinea fowl, rabbit, crab, rose fish, chives and papaya.

## Revendications

1. Utilisation d'un support comprenant au moins une zone d'absorption basée sur une polyoléfine non tissée ou utilisation de la zone d'absorption pour la détermination semi-quantitative, de préférence quantitative de la concentration en un analyte dans un échantillon sanguin.

2. Procédé pour la détection d'un analyte dans un échantillon sanguin, comprenant les étapes de :
a) mise à disposition d'un support comprenant au moins une zone d'absorption basée sur une polyoléfine non tissée,
b) application d'un échantillon de sang capillaire sur la zone d'absorption,
c) séchage du support,
d) mise en contact du support ou de sa zone d'absorption comprenant l'échantillon sanguin séché de l'étape c) avec un liquide approprié pour absorber l'analyte de l'échantillon sanguin séché et
e) détection de l'analyte dans le liquide.

3. Utilisation ou procédé selon la revendication 1 ou 2, ladite au moins une zone d'absorption étant entourée par un matériau présentant une capacité d'absorption inférieure à celle de la zone d'absorption, de préférence du carton-pâte.

4. Utilisation ou procédé selon l'une des revendications 1 à 3, le support comprenant au moins deux zones d'absorption basées sur une polyoléfine non tissée, qui sont séparées l'une de l'autre, de préférence par une zone basée sur un matériau présentant une capacité d'absorption inférieure à celle de la zone d'absorption, de préférence du carton-pâte.

5. Utilisation ou procédé selon l'une des revendications 1 à 4, l'échantillon sanguin étant du sang capillaire.

6. Utilisation ou procédé selon l'une des revendications 1 à 5, le sang capillaire étant du sang capillaire non traité.

7. Utilisation ou procédé selon l'une des revendications 1 à 6, la polyoléfine étant du polypropylène.

8. Utilisation ou procédé selon l'une des revendications 1 à 7, la détection étant une détection semi-quantitative, de préférence une détection quantitative.

9. Utilisation ou procédé selon l'une des revendications 1 à 8, la détection ou la détermination étant réalisée à l'aide d'un support utile en diagnostic comprenant un agent destiné à capturer spécifiquement un anticorps.

10. Utilisation ou procédé selon la revendication 9, le support utile en diagnostic étant choisi dans le groupe constitué par un transfert ; de préférence un transfert en ligne, un transfert en point ou un Westernblot ; une bille, une plaque de microtitrage, une bandelette réactive, une puce à ADN, un support en verre, une biopuce et un dispositif influent latéral.

11. Utilisation ou procédé selon l'une des revendications 1 à 10, l'analyte étant un anticorps, de préférence un anticorps de la classe des IgG.

12. Utilisation ou procédé selon la revendication 11, l'anticorps étant un anticorps contre une matière contenant des allergènes et/ou des protéines, basée sur un aliment, de préférence du groupe comprenant la courgette, le poivron, la viande de volaille, le cabillaud, le saumon, la noix, la pastèque, le kiwi, la pintade, le lapin, le crabe, le sébaste, la ciboulette et la papaye.
